# EUROPEAN PATENT SPECIFICATION

(11) **EP 3 586 955 B1**
(45) Date of publication and mention of the grant of the patent: **22.06.2022**
(21) Application number: 19755511.3
(22) Date of filing: 12.02.2019
(51) Int. Cl.: B01J 20/04, B01J 27/18, B01J 20/28, B01J 35/02, B01J 37/00, B01J 37/03, B01J 20/30, B01D 53/02, C01B 25/32, A61L 9/014, A61L 101/16

(54) **METHOD FOR PRODUCING CALCIUM PHOSPHATE USED IN A HARMFUL GAS- AND ODOR-REMOVING COMPOSITION**
VERFAHREN ZUR HERSTELLUNG VON CALCIUMPHOSPHAT BENUTZT IN EINER ZUSAMMENSETZUNG ZUR ENTFERNUNG VON SCHÄDLICHEM GAS UND GERUCH
PROCÉDÉ DE PRODUCTION DE PHOSPHATE DE CALCIUM UTILISÉ DANS UNE COMPOSITION D'ÉLIMINATION DE GAZ NOCIFS ET D'ODEURS

(30) Priority: 06.03.2018 KR 20180026164; 16.03.2018 KR 20180030844
(43) Date of publication of application: 01.01.2020
(73) Proprietor: Basearth Co., Ltd., Seoul 04147 (KR)
(72) Inventor: KIM, Yong, III, Seoul 04147 (KR); SHIN, Euisip, Seoul 04147 (KR)
(74) Representative: Witte, Weller & Partner Patentanwälte mbB
(86) International application number: PCT/KR2019/001712
(87) International publication number: WO 2019/172544

(56) References cited:
- EP-A1- 1 183 100
- WO-A1-2013/093439
- WO-A1-2015/173437
- JP-A- H03 146 132
- JP-B2- 3 020 784
- JP-B2- 5 224 425
- KR-B1- 100 506 333
- KR-B1- 100 803 110
- KR-B1- 101 809 014
- KR-B1- 101 933 441
- US-A1- 2012 265 152
- VOLKMER. T. M.: "Novel method for the obtainment of nanostructured calcium phosphate cements: Symhesis, mechanical strength and cytmoxicity", Powder technology, vol. 235, 23 October 2013 (2013-10-23), pages 599-605, XP028973452, ISSN: 0032-5910, DOI: 10.1016/j.powtec.2012.10.025
- BLUMENTHAL, N C.: "Effect of preparation conditions on the properties and transfom1ation of amorphous calcimn phosphate", Materials Research Bulletin, vol. 7, no. 11, 1 November 1972 (1972-11-01), pages 1181-1189, XP024076892, ISSN: 0025-5408, DOI: 10.1016/0025-5408(72)90097-9

## Description

### [Technical Field]

The present invention relates to a method for preparing calcium phosphate used in a a composition for removing harmful gas and odor

### [Background Art]

Following the progress of industrialization, the atmospheric environment is gradually getting worse and pollutants are becoming more diverse, and in the present reality, the generation rate of various harmful gases including heavy metals, carbon monoxide (CO), nitrogen oxides (NOₓ) and volatile organic compounds (VOCs) which are critical to the human body is rapidly increasing. Accordingly, with growing interest in the atmospheric environment around the world, highly efficient air filters are being used to obtain a clean living environment and to maintain a highly-clean production process along with the development of electric, electronic, and semiconductor industries.

However, such a high efficiency air filter has a disadvantage in that the selling price is high due to an increase in production cost, and although improvement is possible by the removal of harmful gas, there is a problem in that the removal efficiency is still low.

There are a variety of pollutants in the air ranging from ones in a dust state and ones in a gas state. Among the exhaust gases are neutral gases such as butane, toluene, benzene, and acetaldehyde, and acidic gases such as sulfur dioxide, carbon monoxide, nitrogen oxide, and ozone. Tobacco smoke contains neutral gases such as toluene and acetaldehyde, acidic gases such as acetic acid, and basic gases such as ammonia and the like. The human body also generates carbon dioxide and ammonia, and livestock and such generate methyl sulfide which is a neutral gas, hydrogen sulfide which is an acidic gas and ammonia which is a basic gas.

These pollutants can be removed to some extent by a filter provided in a, an air conditioner, a total heat exchanger, and the like.

However, the filters provided in an air purifier, an air conditioner, a total heat exchanger and the like have high removal efficiency of particulate pollutants and biological pollutants but have a problem of low removal efficiency of gaseous substances.

Meanwhile, hydroxyapatite (HAP) is a calcium phosphate-based ceramic which is a main constituent of human bone and has excellent bone conduction, bioactivity, biocompatibility, protein adsorption, heavy metal adsorption, antibacterial and anti-viral properties and so in general, it is mainly used as a bone substitute in the fields of orthopedics and dentistry.

WO 2013/093439 A1 discloses a calcium phosphate material. US 2012/0265152 A1 discloses a hydroxylapatite material, and a method for the production thereof. WO 2015/173437 A1 discloses a process for producing a calcium phosphate reactant, a reactant obtained and use thereof in the purification of liquid effluents.

KR 101 809 014 B1 discloses a spray composition capable of improving the particulate matter removal efficiency of a filter. JP 5 224425 B2 discloses a coating composition. EP 1 183 100 A1 discloses a mineral salt based substantially or totally hydrosoluble composition formulation containing said composition and the production thereof.

### [Detailed Description of the Disclosure]

### [Technical Problem]

Therefore, the present invention is directed to provide a method for preparing calcium phosphate that can prepare calcium phosphate simpler than a conventional method for preparing calcium phosphate, wherein the prepared calcium phosphate has a large specific surface area so that even with a small amount it has excellent removal efficiency of harmful gases that are harmful to the human body, and is secured with durability.

The present invention is also directed to provide a method for preparing calcium phosphate which can significantly reduce the operation cost as compared with the conventional adsorbent or catalyst for removing harmful gas when calcium phosphate prepared according to the present invention is applied to an industrial or indoor air purifier or the like.

In the present invention, it is also described, but not claimed a composition capable of removing harmful gas and odor by being applied to a filter, a pollution mask or the like.

### [Technical Solution]

In order to solve the above problems, a method for preparing calcium phosphate according to claim 1 is provided. The method of claim 1 comprises steps (a) to (f).

According to an embodiment, it is preferable that the calcium salt is one or more selected from the group consisting of Ca(NO₃)₂, Ca(OH)₂, CaCO₃, Ca(CH₃COO)₂, CaCl₂ and CaSO₄, the phosphate is one or more selected from the group consisting of (NH₄)₂HPO₄, NH₄H₂PO₄, H₃PO₄, H₄P₂O, NaH₂PO₄ and KH₂PO₄, and the carbonate is at least one selected from the group consisting of (NH₄)₂CO₃, NH₄HCO₃, K₂CO₃ and Na₂CO₃.

According to an embodiment, it is preferable that in above-mentioned step (c), the basic solution is added such that the calcium salt solution of above-mentioned step (a) has a pH of 8 to 12, and in above-mentioned step (d), the basic solution is added such that the mixed solution of above-mentioned step (b) has a pH of 8 to 12.

According to an embodiment, it is preferable that the basic solution is one or more selected from the group consisting of NH₄OH, NaOH, KOH, LiOH and Mg(OH)₂.

According to an embodiment, it is preferable that in above-mentioned step (f), the precipitate of above-mentioned step (e) is filtered with a filter, washed with distilled water or an organic solvent, and dried at a temperature of 50 to 100 °C.

### [Advantageous Effects]

According to the present invention, it is provided a method for preparing calcium phosphate used in a composition for removing harmful gas and odor. Calcium phosphate obtained therefrom, can be used as an adsorbent or catalyst capable of removing harmful gases that are harmful to the human body in the air or that are generated in various factories and is superior in performance than a conventional adsorbent or catalyst for removing harmful gas, and is advantageous in that durability and stability can be ensured. In addition, by simply applying by way of spraying on a filter of an air purifier, on an air filter of a car, a pollution mask and such, it is possible to increase the efficiency of the filter and effectively remove odors as well, and the manufacturing process is simple, thereby lowering the manufacturing costs and having an economical effect.

### [Description of the Drawings]

FIG. 1 shows a method for preparing calcium phosphate according to the present invention by way of a flowchart.
FIG. 2 shows a schematic diagram of a self-made test instrument.
FIGS. 3(a) and (b) show images of the self-made test instrument.
FIG. 4 is a graph showing the formaldehyde removal efficiency of Example 1.
FIG. 5 is a graph showing the ammonia removal efficiency of Example 1.
FIG. 6 is a graph comparing the formaldehyde and ammonia removal efficiencies of Example 1.
FIGS. 7(a) and (b) are scanning electron microscope images of calcium phosphate prepared according to Example 1.
FIG. 8 shows the performance tests of the composition prepared according to Comparative Example 3 and Examples 1 to 3 for removing harmful gases.
FIG. 9 shows the performance tests of the composition prepared according to Comparative Example 3, Example 3 and Example 4 for removing harmful gases.
FIG. 10 shows the performance tests for removing harmful gases in Comparative Examples 1 to 3 and Example 4.
FIG. 11 shows an image of a self-made pollution mask test instrument.
FIG. 12 is an image showing a detector tube type gas measurement device of Gastec, which is used for measuring the concentration of harmful gas.
FIG. 13 is an image of a pollution mask sample for performance evaluation.

### [Best Mode for carrying out the Invention]

First, Ca(NO₃)₂ was added to distilled water and stirred at room temperature (25 °C) for 30 minutes to prepare 100 ml of a 1 M Ca(NO₃)₂ solution (first solution).

Next, (NH₄)₂PO₄ was added to distilled water and stirred at room temperature (25 °C) for 30 minutes to prepare 100 ml of a 0.6 M (NH₄)₂HPO₄ solution (second solution) and (NH₄)₂CO₃ was added to distilled water and stirred at room temperature (25 °C) for 30 minutes to prepare 100 ml of a 0.3 M (NH₄)₂CO₃ solution (third solution).

The prepared 0.6 M (NH₄)₂HPO₄ solution (second solution) and 0.3 M (NH₄)₂CO₃ solution (third solution) were rapidly mixed and then NH₄OH was added dropwise to adjust the pH to 10. NH₄OH was added dropwise to 1 M Ca(NO₃)₂ solution (first solution) to adjust the pH to 10.

Thereafter, the mixed solution of the two solutions and 1 M Ca(NO₃)₂ solution were rapidly mixed and stirred at room temperature (25 °C) for 30 minutes to prepare a precipitate.

Finally, the precipitate was filtered with a filter, washed with distilled water and ethyl alcohol, and dried at 60 °C for 12 hours to complete the preparation of calcium phosphate.

### [Modes for carrying out the Invention]

Hereinafter, a preferable exemplary embodiment of the present invention will be described in detail with reference to the accompanying drawings.

The advantages and features of the present invention and the manner of accomplishing it will become apparent with reference to the embodiments described in detail below with reference to the accompanying drawings.

Rather, these embodiments are provided so that this disclosure will be thorough and complete, and will fully disclose the present invention and to fully convey the scope of the invention to those skilled in the art. In addition, the invention is only defined by the scope of the claims.

Further, in the following description of the present invention, if it is determined that related arts or the like may obscure the gist of the present invention, detailed description thereof will be omitted.

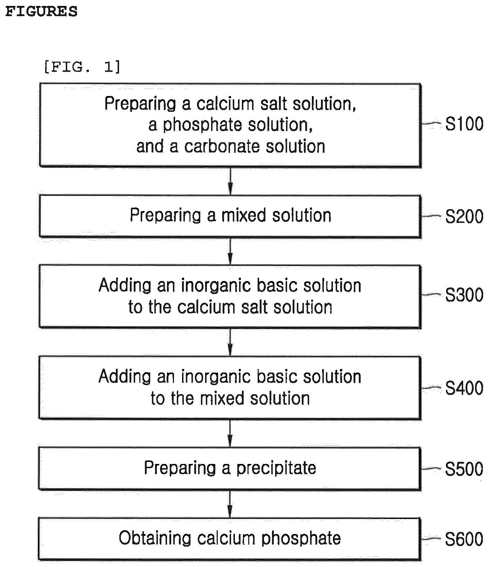

Hereinafter, a method for preparing calcium phosphate according to the present invention will be described with reference to FIG. 1.

FIG. 1 is a flowchart illustrating a method for preparing calcium phosphate according to the present invention.

The method for preparing calcium phosphate according to the present invention is characterized in that the method includes, (a) preparing a calcium salt solution, a phosphate solution, and a carbonate solution; (b) mixing the phosphate solution and the carbonate solution of the above-mentioned step (a) to prepare a mixed solution; (c) adding a basic solution into the calcium salt solution of the above-mentioned step (a); (d) adding a basic solution into the mixed solution of the above-mentioned step (b); (e) mixing and reacting the calcium salt solution into which the basic solution has been added in the above-mentioned step (c) with the mixed solution into which the basic solution has been added in the above-mentioned step (d) to prepare a precipitate; and (f) filtering, washing and drying the precipitate of the above-mentioned step (e) to obtain calcium phosphate.

In the method for preparing calcium phosphate according to the present invention, the above-mentioned step (a) in S100 is a step of preparing a calcium salt solution, a phosphate solution, and a carbonate solution.

The calcium salt solution may be prepared by mixing calcium salt and distilled water at a temperature of room temperature (22 to 30 °C) for 10 to 60 minutes using a stirrer, and more specifically, by mixing them for 30 minutes. The concentration of the calcium salt solution is 0.5 to 1.5 M, and specifically may be 1 M. The concentration range of the calcium salt solution is for preparing calcium phosphate having a high specific surface area, and so in the case where the concentration is out of the above range, it affects the properties of the final product.

Further, the phosphate solution and the carbonate solution are also prepared by the same method as the calcium salt solution, and the description of the preparation method is omitted.

The concentration of the phosphate solution is 0.3 to 0.8 M, and specifically may be 0.6 M. The concentration range of the calcium salt solution is for preparing calcium phosphate having a high specific surface area, and so in the case where the concentration is out of the above range, it affects the properties of the final product.

The concentration of the carbonate solution is 0.1 to 0.5 M, and specifically may be 0.3 M. The concentration range of the carbonate solution is for preparing calcium phosphate having a high specific surface area, and so in the case where the concentration is out of the above range, it affects the properties of the final product.

The calcium salt may be one or more selected from the group consisting of Ca(NO₃)₂, Ca(OH)₂, CaCO₃, Ca(CH₃COO)₂, CaCl₂ and CaSO₄, and specifically may be Ca(NO₃)₂.

The phosphate may be one or more selected from the group consisting of (NH₄)₂HPO₄, NH₄H₂PO₄, H₃PO₄, H₄P₂O, NaH₂PO₄ and KH₂PO₄, and specifically may be (NH₄)₂HPO₄.

The carbonate may be one or more selected from the group consisting of (NH₄)₂CO₃, NH₄HCO₃, K₂CO₃ and Na₂CO₃, and specifically may be (NH₄)₂CO₃.

In the method of preparing calcium phosphate according to the present invention, the above-mentioned step (b) in S200 is a step of mixing a phosphate solution and a carbonate solution of the above-mentioned step (a) in S100.

The step of preparing the mixed solution of the above-mentioned step (b) in S200 is not limited as long as it is a method capable of rapid mixing.

In the method for preparing calcium phosphate according to the present invention, the above-mentioned step (c) in S300 is a step of adding a basic solution into the Ca(NO₃)₂ solution of the above-mentioned step (a) in S100.

In the above-mentioned step (c) in S300, the basic solution may be added such that the calcium salt solution of the above-mentioned step (a) in S100 has a pH of 8 to 12, and specifically, may be added such that the calcium salt solution of the above-mentioned step (a) in S100 has a pH of 10.

If the pH of the calcium salt solution exceeds 12, Ca ions are re-precipitated in the form of Ca(OH)₂, which affects the properties of the final product, and therefore the above range is preferable.

In the method of preparing calcium phosphate according to the present invention, the above-mentioned step (d) in S400 is a step of adding a basic solution into the mixed solution of the above-mentioned step (b) in S200.

In the above-mentioned step (d) in S400, the basic solution may be added such that the mixed solution of the above-mentioned step (b) in S200 has a pH of 8 to 12, and specifically, may be added such that the mixed solution of the above-mentioned step (b) in S200 has a pH of 10. In the case where the pH of the mixed solution in which the phosphate solution and the carbonate solution are mixed is out of the above range, it affects the properties of the final product. Therefore, the above range is preferable. The pH of the Ca(NO₃)₂ solution of the above-mentioned step (c) in S300 and the mixed solution of the above-mentioned step (d) in S400 can be measured using a pH meter, and a description thereof will be omitted.

In the method for preparing calcium phosphate according to the present invention, the above-mentioned step (e) in S500 is a step of mixing and reacting the calcium salt solution into which the basic solution has been added in the above-mentioned step (c) in S300 with the mixed solution into which the basic solution has been added in the above-mentioned step (d) in S400 to prepare a precipitate.

Here, the precipitate may be precipitated calcium phosphate.

The basic solution may be one or more selected from the group consisting of NH₄OH, NaOH, KOH, LiOH, and Mg (OH)₂, and may specifically be NH₄OH.

The above-mentioned step (e) in S500 may be performed using a stirrer for 10 to 120 minutes and specifically, may be performed by using a stirrer for 30 minutes.

In the method for preparing calcium phosphate according to the present invention, the above-mentioned step (f) in S600 is a step of filtering, washing and drying the precipitate of the above-mentioned step (e) in S500 to obtain calcium phosphate.

In the above-mentioned step (f) in S600, the precipitate of the above-mentioned step (e) in S500 may be filtered with a filter, washed with distilled water or an organic solvent, and dried at a temperature of 50 to 100 °C.

The filtering of the precipitate in the above-mentioned step (e) in S500 is for removing residue remaining in the solution after the reaction of step (e) in S500 and for recovering the generated calcium phosphate, but not limited as long as it is a method capable of obtaining calcium phosphate generated in the solution.

The precipitate of step (e) in S500 may be filtered and then washed with distilled water or organic solvent, wherein the precipitate may be washed repeatedly 2 to 3 times using the distilled water or the organic solvent, may be redispersed in the organic solvent, or may be washed alternately between distilled water and organic solvent, but the method is not limited.

The organic solvent is not limited as long as it can wash the precipitate, such as ethyl alcohol, methyl alcohol and the like.

After washing the precipitate, the washed precipitate may be dried at a temperature of 50 to 100 °C for 10 to 24 hours. Specifically the precipitate may be dried at a temperature of 60 °C for 12 hours. If the drying temperature is less than 50 °C, the precipitate may not be dried well, and if the drying temperature exceeds 100 °C, discoloration and property change of the precipitate may occur. Therefore, the above range is preferable.

The precipitate obtained in the above-mentioned step (f) in S600 is calcium phosphate, and the specific surface area is from 40 to 150 m²/g. In addition, the primary particle size of the calcium phosphate is 100 nm or less, and the primary particles may be in an aggregated form. Specifically, the size of the primary particle may be 1 to 100 nm.

The harmful gas to be removed by using the calcium phosphate prepared according to the method of the present invention may be one or more selected from the group consisting of nitrogen oxide (NOₓ), volatile organic compounds (VOCs), carbon monoxide (CO), ammonia (NH₃), and the substance capable of generating odor may include methyl sulfide, hydrogen sulfide, methyl disulfide, methyl sulfide trimethyl amine, sulfurous acid, and the like.

Calcium phosphate prepared according to the present invention may be provided.

The primary particle size of the calcium phosphate is 100 nm or less, and the primary particles may be in an aggregated form. Specifically, the size of the primary particle may be 1 to 100 nm.

The calcium phosphate has a specific surface area ranging from 40 to 150 m²/g.

The calcium phosphate may have a harmful gas removal efficiency of 78 to 99.8%.

In the present invention, the primary particle means calcium phosphate.

Described herein, but not claimed is a composition for removing harmful gas and odor.

The composition for removing harmful gas and odor is characterized in that the composition contains calcium phosphate as an active ingredient.

Specifically, the calcium phosphate may be hydroxyapatite.

The hydroxyapatite may be several nanometers to several tens of nanometers in size and may have a specific surface area of 40 m²/g or more.

The hydroxyapatite is a kind of calcium phosphate and is used as a substitute for a hard tissue of a living body such as artificial root for teeth or bone filler. A single crystal-like rod-type hydroxyapatite is used for increasing the fracture strength of an artificial bone. In addition, since the hydroxyapatite has both positive (+) and negative (-) charged surfaces of the crystal, it is possible to significantly increase the adsorbing ability, and thus has excellent deodorizing effects and is also used for soap and natural inorganic antibacterial agents. Further, other than the co-precipitation method, the hydroxyapatite may be synthesized by a hydrothermal synthesis method or a solid-phase reaction method, and a technique for controlling the aspect ratio of apatite and a technique for inducing defects on the crystal structure is applied to control the area and surface polarity intensity of the positive (+) and negative (-) charged surfaces, thereby it is possible to improve the adsorption ability of a target.

The hydroxyapatite is a solid component, and based on 100 wt% of a composition according to an embodiment of the present invention, the solid component may include 0.1 to 10 wt%, and when the solid content is out of the above range, the solid content cannot be uniformly dispersed in the composition, and the efficiency of the initial fractionation removal of the filter or pollution mask can be hindered.

The composition for removing harmful gas and odor may further include a binder, a carrier solution and a surfactant, and the content of the carrier solution may vary depending on the content of calcium phosphate in the composition.

Specifically, based on 100 wt% of the total composition, the calcium phosphate, the binder, the carrier solution, and the surfactant may be included in an amount of 0.1 to 10 wt% of calcium phosphate, 0.5 to 20 wt% of the binder, 69 to 99.35 wt% of the carrier solution and 0.05 to 1 wt% of the surfactant.

The calcium phosphate may specifically be hydroxyapatite and may be in powder form.

If the amount of the calcium phosphate is less than 0.1 wt%, the removal efficiency of harmful gas may deteriorate. If the amount of the calcium phosphate exceeds 10 wt%, there is a problem in that dust may be generated because after the composition is applied to the filter and dried, a complete adherence is not made, and therefore the above range is preferable.

If the carrier solution is less than 69 wt%, the hydroxyapatite is not sufficiently dispersed, and spraying cannot be performed uniformly. When the carrier solution exceeds 99.35 wt%, there is a problem in that the content of the binder is too low, so when applied to a filter or pollution mask, it fails to adhere sufficiently to the surfaces and the powder falls off. Therefore, the above range is preferable.

The carrier solution may be one or more selected from the group consisting of water, ethanol, methanol, ethyl acetate, butanol, propylene glycol, butylene glycol, and glycerin. Specifically, it may be water, and may be mixed according to the use environment and conditions.

If the amount of the binder is less than 0.5 wt%, there is a problem in that a solid component as an adsorbent in the composition may not adhere to the filter surface. If the binder exceeds 20 wt%, the viscosity of the binder may be too high to disperse calcium phosphate in the composition and fails to be sprayed uniformly. Therefore, the above range is preferable.

The binder may be one or more selected from the group consisting of polyvinyl alcohol, alginic acid, gelatin, cellulose and derivatives thereof, and may be specifically polyvinyl alcohol. The binder may be a solid component or may be used in a state dissolved in a solvent. In the case the binder is dissolved in a solvent, the solvent is included in the weight percentage of the organic solvent.

The surfactant may be a polyoxyalkylene-based surfactant and is not limited if it serves as a de-foaming agent and enhances the stability of dispersion.

If the amount of the surfactant is less than 0.05 wt%, the de-foaming function cannot be performed and so foaming may occur. If the amount of the surfactant exceeds 1 wt%, it may affect the adsorbing property of the raw material and is not economically preferable. Therefore, the above range is preferable.

The composition for removing harmful gas and odor may further include additives, a bactericide, and a perfume component that can improve the harmful gas removal efficiency in addition to the composition components described above. The % by weight of the further added components may be subtracted from the % by weight of the carrier solution, and the kind thereof is not limited.

The harmful gas to be removed may be one or more selected from the group consisting of nitrogen oxide (NOₓ), volatile organic compounds (VOCs), carbon monoxide (CO), and ammonia (NH₃), and the substance capable of generating odor may include methyl sulfide, hydrogen sulfide, methyl disulfide, methyl sulfide trimethyl amine, sulfurous acid, and the like.

The composition for removing harmful gases and odors can remove 10% or more of one or more harmful gases selected from the group consisting of nitrogen oxide (NOₓ), volatile organic compounds VOCs, carbon monoxide (CO), and ammonia (NH₃) and can remove 10 to 100 % of harmful gas and odor according to environment, conditions and material.

The composition for removing harmful gas and odor described herein may be prepared by using the above-mentioned calcium phosphate, water, polyvinyl alcohol and surfactant using a mechanical stirrer, and the stirring temperature and stirring time are not limited.

A spray for removing harmful gas and odor may be provided, characterized in that the spray includes a case for spraying and a composition to be filled in the case, and the composition, includes a case for spraying and a composition to be filled in the case, and based on 100 wt% of the total composition, the composition includes 0.1 to 10 wt% of calcium phosphate, 0.5 to 20 wt% of a binder, 69 to 99.35 wt% of a carrier solution and 0.05 to 1 wt% of a surfactant.

The case for spraying is not limited to a generally used spraying device, and when spraying a large amount on a filter, the composition may be sprayed using a spray gun connected to a high-capacity compressor. Further, the viscosity and the weight ratio of the composition are described above, and a description thereof will be omitted.

The composition for removing harmful gas and odor may be applied by the above-described spray, or may be spray-applied using a spray gun connected to a compressor, but is not limited thereto, and the type is not limited as long as it can be applied in liquid form.

Hereinafter, the present invention will be described in more detail with reference to specific examples.

### Example 1: Preparation of calcium phosphate

First, Ca(NO₃)₂ was added to distilled water and stirred at room temperature (25 °C) for 30 minutes to prepare 100 ml of a 1 M Ca(NO₃)₂ solution (first solution).

Next, (NH₄)₂PO₄ was added to distilled water and stirred at room temperature (25 °C) for 30 minutes to prepare 100 ml of a 0.6 M (NH₄)₂HPO₄ solution (second solution) and then (NH₄)₂CO₃ was added to distilled water and stirred at room temperature (25 °C) for 30 minutes to prepare 100 ml of a 0.3 M (NH₄)₂CO₃ solution (third solution).

The prepared 0.6 M (NH₄)₂HPO₄ solution (second solution) and 0.3 M (NH₄)₂CO₃ solution (third solution) were rapidly mixed and then NH₄OH was added dropwise to adjust the pH to 10. NH₄OH was also added dropwise to 1 M Ca(NO₃)₂ solution (first solution) to adjust the pH to 10.

Thereafter, the mixed solution of the two solutions and 1 M Ca(NO₃)₂ solution were rapidly mixed and stirred at room temperature (25 °C) for 30 minutes to prepare a precipitate.

Finally, the precipitate was filtered with a filter, washed with distilled water and ethyl alcohol, and dried at 60 °C for 12 hours to complete the preparation of calcium phosphate.

### Example 2: Composition for removing harmful gas and odor

10 g of the calcium phosphate powder prepared according to Example 1 and 1 g of a surfactant were prepared.

600 ml of a solution of polyvinyl alcohol (5 wt%) and 389 ml of water were mixed and prepared.

Finally, the prepared raw materials were placed in a mechanical stirrer and stirred at 25 °C to complete the preparation of the composition.

### Example 3: Composition for removing harmful gas and odor

A composition was prepared in the same manner as in Example 2 except that 20 g of calcium phosphate powder and 379 ml of water were added in place of 10 g of calcium phosphate powder and 389 ml of water.

### Example 4: Composition for removing harmful gas and odor

A composition was prepared in the same manner as in Example 2 except that 30 g of calcium phosphate powder and 369 ml of water were added in place of 10 g of calcium phosphate powder and 389 ml of water.

### Example 5:Composition for removing harmful gas and odor

A composition was prepared in the same manner as in Example 4 except that 319 ml of water, 40 ml of natural cypress water, and 10 ml of natural cypress oil were added in place of 369 ml of water.

### Comparative Example 1

A composition was prepared in the same manner as in Example 4, except that a commercially available A-type calcium phosphate powder of C company was used instead of the synthesized calcium phosphate powder.

### Comparative Example 2

The composition was prepared in the same manner as in Example 4, except that a commercially available B-type calcium phosphate powder of C company was used instead of the synthesized calcium phosphate powder.

### Comparative Example 3

A commercially available pollution mask was purchased and prepared.

### Making of Test Equipment

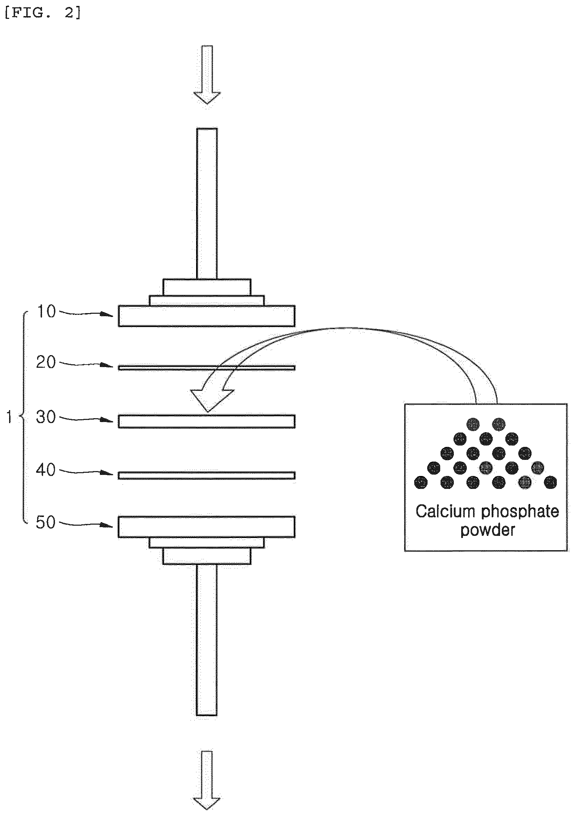

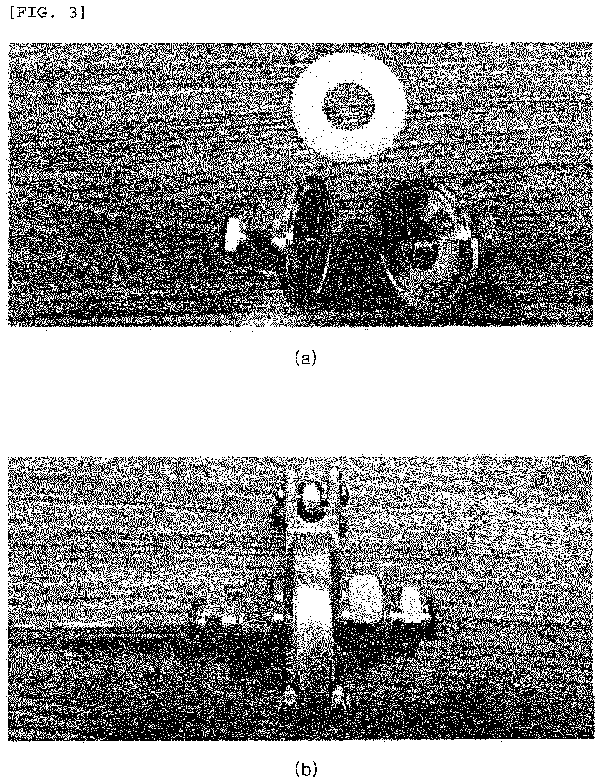

To confirm the removal of harmful gas of the calcium phosphate prepared according to Example 1, a self-made test instrument was made as shown in FIGS. 2 and 3.

FIG. 2 is a schematic diagram of the self-made test instrument, and FIGS. 3(a) and 3(b) show images of a self-made test instrument.

Briefly described, the test instrument 1 is configured to have an upper gasket adapter 10, a cover filter 20, a gasket 30, a circular hole 40 in the gasket, a lower filter and a pipe gasket adapter 50 which are coupled using a clamp, and calcium phosphate may be placed in the circular hole 40 inside the gasket to be used.

The operating principle of the test apparatus is that calcium phosphate is placed in the circular hole 40 in the gasket and then a Tedlar bag in which the harmful gas is collected is injected into the upper gasket adapter 10 and the gas discharged to the pipe gasket adapter 50 of a lower part is recollected and the concentration of the harmful gas can be measured. Harmful gas is sucked in by using a large-sized syringe of 500 ml or more with the pipe gasket adapter 50 of the lower part. The harmful gas collected in the syringe is transferred to a Tedlar gas sampling bag and the concentration can be measured with a harmful gas concentration meter.

A gas sampler (GV-100S) manufactured by Gastec was used for measurement of harmful gas concentration using a tube type gas detector.

(No. 91L was used as the detector tube for measuring formaldehyde (HCHO) gas, and No. 3L was used as the detector tube for measuring ammonia (NH₃) gas.)

### Experimental Example 1: Calcium phosphate performance evaluation

Formaldehyde was used as the harmful gas. The formaldehyde gas was prepared by vaporizing a formaldehyde solution and collecting it in a Tedlar gas sampling bag. The initial concentration of formaldehyde gas was set to 32 ppm.

In order to measure the concentration change of the harmful gas according to the above test equipment, the formaldehyde gas (concentration: 32 ppm) was injected and then the formaldehyde was collected to measure the concentration.

In addition, 0.1 g of calcium phosphate prepared according to Example 1 was placed in the circular hole in the gasket, and the formaldehyde gas was added to measure the ability of calcium phosphate to remove the formaldehyde gas.

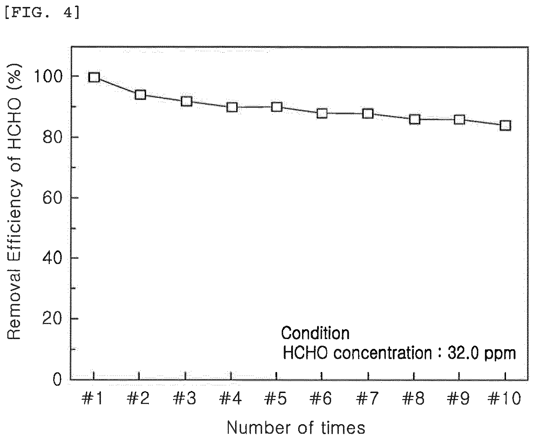

The above procedure (measurement of the ability of calcium phosphate to remove formaldehyde by adding gas) was repeated 10 times and shown in the following Table 1 and FIG. 4. Afterwards, it was left for 30 minutes in the absence of formaldehyde and air flow and measured repeatedly 10 times. It was left for 3 days and then measured once more. The results are shown in Table 2 below.

FIG. 4 is a graph showing the removal efficiency of formaldehyde of Example 1.

**[Table 1]**

| Performance Evaluation of Calcium Phosphate Formaldehyde | | | |
|---|---|---|---|
| Harmful Gas Number of Times | | Formaldehyde (HCHO ) | |
| | | Detected Concentration(pp m) | Removal Efficiency (%) |
| Raw Gas | | 32 | - |
| Formaldehyde Concentration according to Test Equipment | | 24.8 | 0 |
| Example 1 (0.1 g) | 1st Time | <0.05 | 99.80 |
| | 2nd Time | 1.5 | 93.95 |
| | 3rd Time | 2 | 91.94 |
| | 4th Time | 2.5 | 89.92 |
| | 5th Time | 3.0 | 89.92 |
| | 6th Time | 3.0 | 87.90 |
| | 7th Time | 3.0 | 87.90 |
| | 8th Time | 3.5 | 85.89 |
| | 9th Time | 3.5 | 85.89 |
| | 10th Time | 4.0 | 83.87 |

**[Table 2]**

| Performance Evaluation of Calcium Phosphate after leaving for 30 minutes and 3 days | | | |
|---|---|---|---|
| Harmful Gas Number of Times | | Formaldehyde (HCHO) | |
| | | Detected Concentration ( ppm) | Removal Efficiency (%) |
| Raw Gas | | 32 | - |
| Formaldehyde Concentration according to Test Equipment | | 24.8 | 0 |
| Example 1 (0.1 g) after leaving for 30 minutes | 1st Time | 2.5 | 89.92 |
| | 2nd Time | 3.0 | 97.90 |
| | 3rd Time | 3.5 | 85.89 |
| | 4th Time | 3.5 | 85.89 |
| | 5th Time | 4.0 | 83.87 |
| | 6th Time | 4.0 | 83.87 |
| | 7th Time | 4.5 | 81.85 |
| | 8th Time | 4.5 | 81.85 |
| | 9th Time | 5.0 | 79.84 |
| | 10th Time | 5.0 | 79.84 |
| After leaving for 3 days | 1st Time | 0.5 | 97.98 |

Referring to Tables 1, 2 and FIG. 4, it can be confirmed that the initial formaldehyde removal efficiency of the calcium phosphate prepared according to Example 1 is 99.80%, and especially even after continuously measuring 10 times (1st time 99.8%, 10th time 83.8%) shows that the formaldehyde removal efficiency is 80% or more. Further, after measuring 10 times, it was left for 30 minutes and re-measured continuously 10 times and still showed a high removal efficiency of 79.84% (1st time 89.92%, 10th time 79.84%).

In addition, the calcium phosphate prepared according to Example 1 is more effective because after the measurement of the 10th time (83.87%), as it can be confirmed from the results of the 1st time of remeasuring (89.92%) after being left for 30 minutes, the formaldehyde removal efficiency is improved, and it was also confirmed that after leaving for 3 days, the formaldehyde removal efficiency (97.98%) also improved.

The above results show that the calcium phosphate prepared according to Example 1(repetition of using and not using) is excellent in formaldehyde removal efficiency and has excellent durability and stability.

### Experimental Example 2: Calcium phosphate performance evaluation

Ammonia was used as the harmful gas. The ammonia gas was prepared by vaporizing the ammonia solution and collecting it in a Tedlar gas sampling bag. The concentration of the ammonia gas was set to 30 ppm.

In order to measure the concentration change of the ammonia gas according to the above test equipment, the ammonia gas (concentration: 30 ppm) was added and the ammonia gas was collected to measure the concentration.

Further, 0.1 g of calcium phosphate prepared according to Example 1 was placed in the circular hole in the gasket, and initial ammonia gas was added to measure the ability of calcium phosphate to remove ammonia gas.

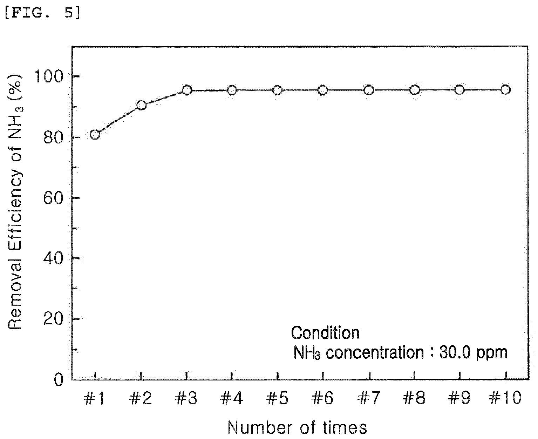

The above procedure was repeated 10 times and then left for 30 minutes in the absence of ammonia gas and air flow. The measurement was repeated once and is shown in Table 3 and FIG. 5 below.

FIG. 5 shows the removal efficiency of ammonia of Example 1.

**[Table 3]**

| Performance Evaluation of Calcium Phosphate for Ammonia | | | |
|---|---|---|---|
| Harmful Gas Number of Times | | Ammonia (NH₃) | |
| | | Detected Concentration (ppm) | Removal Efficiency (%) |
| Raw Gas | | 30 | - |
| Ammonia Concentration according to Test Equipment | | 21 | 0 |
| Example 1(0.1 g) | 1st Time | 4 | 80.95 |
| | 2nd Time | 2 | 90.48 |
| | 3rd Time | 1 | 95.24 |
| | 4th Time | 1 | 95.24 |
| | 5th Time | 1 | 95.24 |
| | 6th Time | 1 | 95.24 |
| | 7th Time | 1 | 95.24 |
| | 8th Time | 1 | 95.24 |
| | 9th Time | 1 | 95.24 |
| | 10th Time | 1 | 95.24 |
| After leaving for 30 minutes | 1st Time | 1 | 95.24 |

Referring to Table 3 and FIG. 5, it can be confirmed that the initial ammonia removal efficiency of the calcium phosphate prepared according to Example 1 was 80.95%, and especially even after continuously measuring 10 times (1st time 80.95%, 10th time 95.24%) shows that the ammonia removal efficiency is 80.95% or more and is maintained Further, after measuring 10 times, it was left for 30 minutes and re-measured continuously 10 times and still showed a high removal efficiency of 95.24% and it can be seen that the efficiency is maintained.

The reason the ammonia removal efficiency improves starting from the 3rd time more than the 1st time and the 2nd time is because the calcium phosphate located on the test equipment is not uniform at first but becomes more uniform as the experiment is repeated, so it can be seen that the efficiency is maintained.

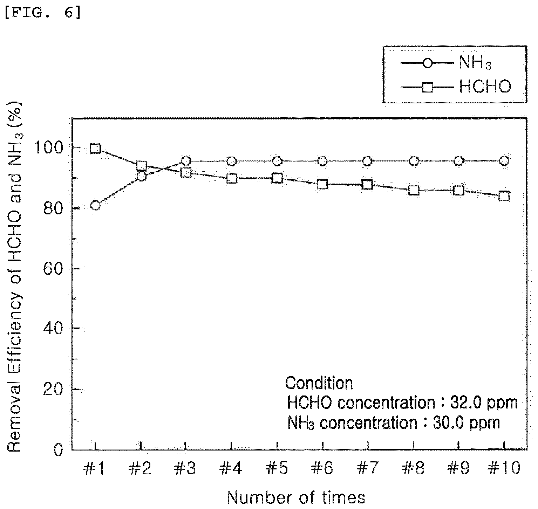

FIG. 6 is a graph comparing the removal efficiency of formaldehyde and ammonia in Example 1.

The above results show that the calcium phosphate prepared according to Example 1(repetition of using and not using) is excellent in ammonia removal efficiency and has excellent durability.

### Experimental Example 3: Measurement of Particle Size and Specific Surface Area of Calcium Phosphate

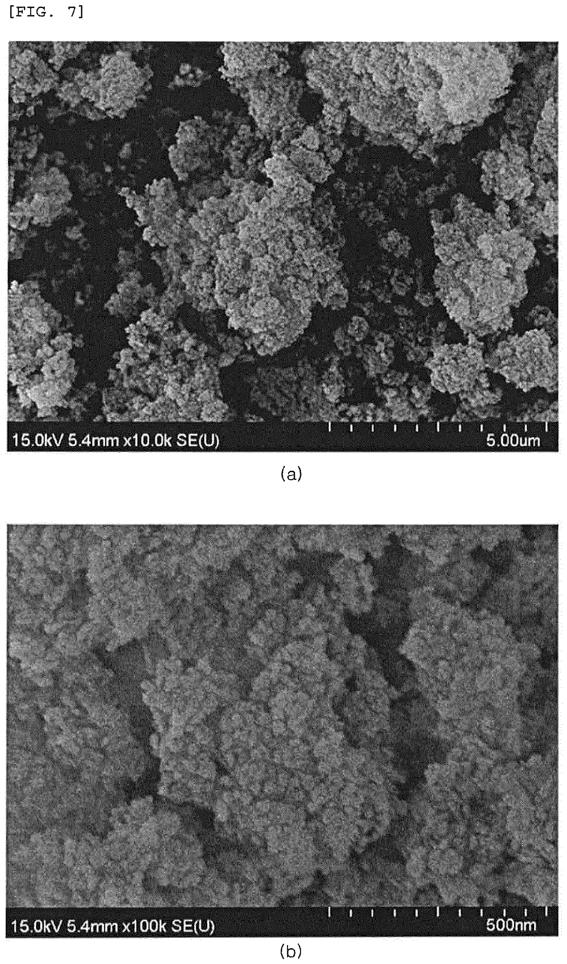

FIGS. 7(a) and 7(b) are scanning electron microscope images of calcium phosphate prepared according to Example 1.

It was confirmed that the calcium phosphate prepared according to Example 1 had a form of aggregated particles of 100 nm or less and it can be confirmed that the calcium phosphate prepared according to Example 1 has an excellent removal efficiency of harmful gas, resulting from being configured of fine particles.

The calcium phosphate prepared according to Example 1 was measured for particle size and specific surface area by a BET measurement method.

The specific surface area (SSA) of calcium phosphate prepared according to Example 1 was found to be 134.3949 m²/g. It can be confirmed that the calcium phosphate prepared according to Example 1 had an excellent removal efficiency of harmful gas, resulting from a large specific surface area.

### Experimental Example 4: Performance Evaluation of Composition for Removing Harmful Gas and Odor

The compositions prepared according to Examples 2 to Comparative Example 2 of the present invention were applied to a pollution mask to evaluate the performance.

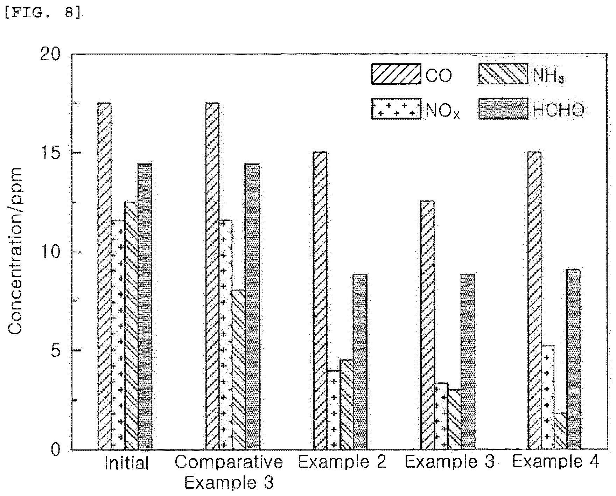

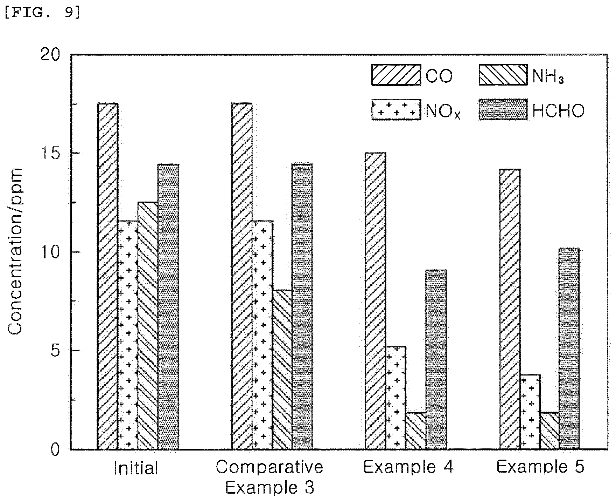

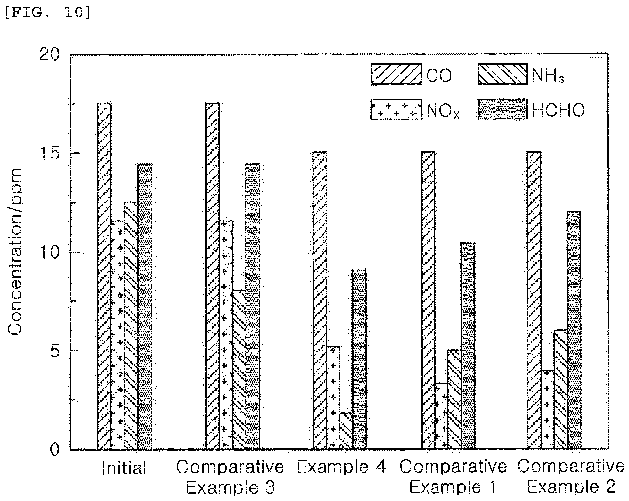

FIG. 8 shows the performance tests of the compositions prepared according to Comparative Example 3 and Examples 2 to 4 for removing harmful gases. FIG. 9 shows the harmful gas removal efficiency test of compositions prepared according to Comparative Example 3, Examples 4 and Example 5, and FIG. 10 shows a harmful gas removal performance test of Comparative Example 1 to 3 and Example 4.

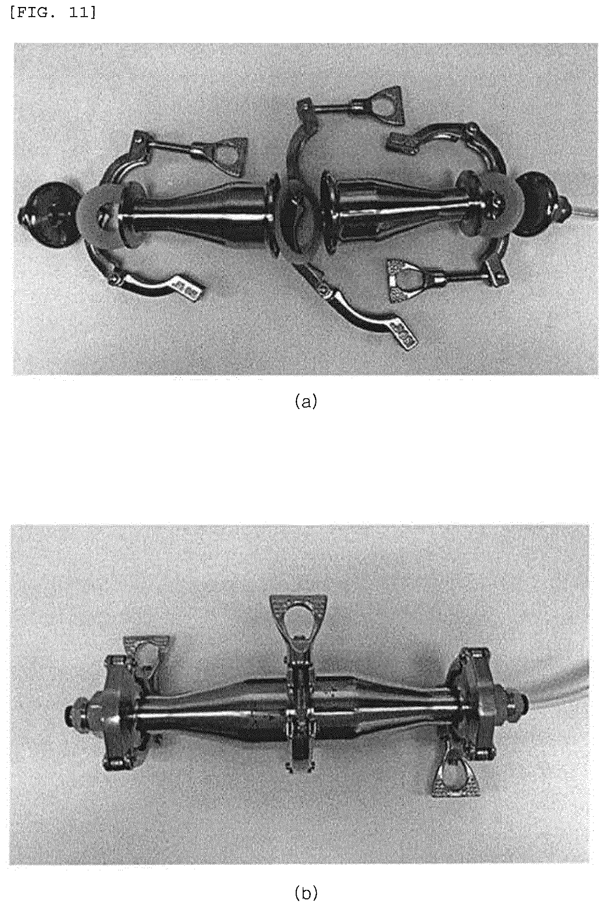

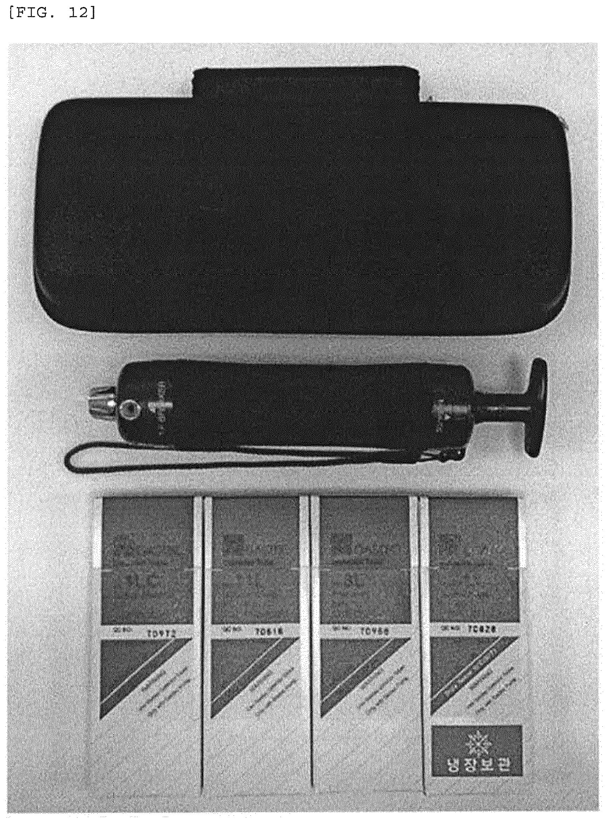

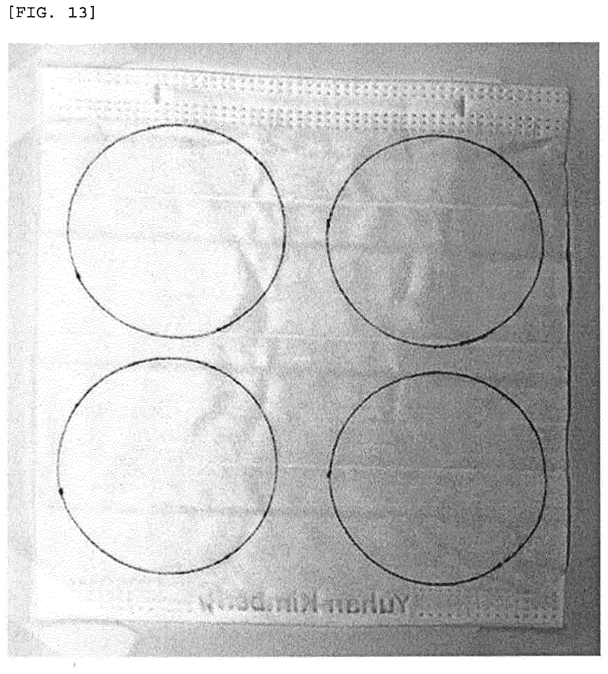

FIG. 11 shows an image of a self-made pollution mask test instrument, and FIG. 12 is an image showing a detector tube type gas detector of Gastec, which is used for measurement of harmful gas concentration, and FIG. 13 shows an image of a pollution mask sample for performance evaluation.

The compositions prepared according to Example 2 to Comparative Example 2 were sprayed on each of the same pollution masks 20 times about 3 ml, and then naturally dried at room temperature for 90 minutes.

Carbon monoxide and nitrogen oxides were prepared by collecting and diluting exhaust gas from a BMW company diesel vehicle that meets the euro6 standard.

The formaldehyde and ammonia gases were prepared by vaporizing the formaldehyde solution and the ammonia solution and collecting them in a Tedlar gas sampling bag.

A commercially available pollution mask was fixed on a self-made pollution mask test instrument, and the performance evaluation was performed.

Here, in the test apparatus, the gasket and the clamp were used to prevent a leak from occurring in the pollution mask.

The gas concentration was measured using a Gastec's gas sampler (GV-100S) equipped with tube type gas detector.

(No. 91L was used as the detector tube for measuring formaldehyde (HCHO) gas, No. 3L was used as the detector tube for measuring ammonia (NH₃) gas, No. 1LC was used as the detector tube for measuring carbon monoxide (CO) and No.11L was used as the detector tube for measuring nitrogen oxide (NO + NO₂).

Tables 4 and 5 below show the compositions prepared according to Examples 2 to Comparative Example 2 applied to a pollution mask and the ability of the pollution mask to remove harmful gases.

**[Table 4]**

| Pollution Mask Performance Test Results | | | | |
|---|---|---|---|---|
| Harmful Gas (unit: ppm) sample | CO | NO + NO₂ | NH₃ | HCHO |
| Initial Concentration | 17.5 | 11.55 | 12.5 | 14.4 |
| Example 1 | 15 | 3.96 | 4.5 | 8.8 |
| Example 2 | 12.5 | 3.3 | 3 | 8.8 |
| Example 3 | 15 | 5.17 | 1.83 | 9.07 |
| Example 4 | 14.17 | 3.74 | 1.83 | 10.13 |
| Comparative Example 1 | 15 | 3.3 | 5 | 10.4 |
| Comparative Example 2 | 15 | 3.96 | 6 | 12 |
| Comparative Example 3 | 17.5 | 11.55 | 8.5 | 14.4 |

**[Table 5]**

| Harmful Gas Removal Efficiency | | | | |
|---|---|---|---|---|
| Harmful Gas (unit: ppm) sample | CO | NO + NO₂ | NH₃ | HCHO |
| Initial Concentration | - | - | - | - |
| Example 1 | 14.3 | 65.7 | 64.0 | 38.9 |
| Example 2 | 28.6 | 71.4 | 76.0 | 38.9 |
| Example 3 | 14.3 | 55.2 | 85.4 | 37.0 |
| Example 4 | 19.0 | 67.6 | 85.4 | 29.7 |
| Comparative Example 1 | 14.3 | 71.4 | 60.0 | 27.8 |
| Comparative Example 2 | 14.3 | 65.7 | 52.0 | 16.7 |
| Comparative Example 3 | 0 | 0 | 36.0 | 0 |

Referring to Tables 4 and 5 and FIGS. 8 to 10, it was confirmed that only the ammonia was removed by 36% in Comparative Example 3 (commercially available pollution mask), but other harmful gases could not be removed. In addition, Comparative Examples 1 and 2 show higher harmful gas removal efficiencies of carbon monoxide (CO), nitrogen oxide (NO + NO₂), ammonia (NH₃) and formaldehyde (HCHO) than those of Comparative Example 3, but it can be confirmed that each of the harmful gases cannot be removed uniformly. On the other hand, it can be confirmed that Examples 2 to 5 can remove each of the harmful gases with high efficiency. Particularly in Example 3, high harmful gas removal efficiencies can be seen for carbon monoxide 28.6%, nitrogen oxide 71.4%, ammonia 76.0% and formaldehyde 38.9%.

Based on the above test results, it was confirmed that the composition containing calcium phosphate prepared according to the present invention has excellent characteristics of removing harmful gas and odor.

Although the method for preparing calcium phosphate according to the present invention and the composition for removing harmful gas and odor including calcium phosphate obtained by the method have been described above, it will be apparent that various modifications may be made without departing from the scope of the present invention.

Therefore, the scope of the present invention should not be limited by the described embodiments but should be determined by the appended claims.

That is, it is to be understood that the foregoing embodiments are illustrative and not restrictive in all respects and that the scope of the present invention is indicated by the appended claims rather than the foregoing description.

### [Industrial applicability]

The method for preparing calcium phosphate according to the present invention and the composition for removing harmful gas and odor containing calcium phosphate obtained therefrom can be used as an adsorbent or catalyst capable of removing harmful gases that are harmful to the human body in the air or that are generated in various factories, and is superior in performance compared to a conventional adsorbent or catalyst for removing harmful gas, and is advantageous in that durability and stability can be ensured. In addition, by simply applying by way of spraying on a filter of an air purifier used indoors, a filter of an air conditioner, an air filter of a vehicle, a pollution mask and such, it is possible to increase the efficiency of the filter and effectively remove odors as well, and the manufacturing process is simple, thereby lowering the manufacturing costs and having an economical effect.

## Claims

1. A method for preparing calcium phosphate **characterized in that** the method comprises,
(a) preparing a calcium salt solution of 0.5 to 1.5 M, a phosphate solution of 0.3 to 0.8 M, and a carbonate solution of 0.1 to 0.5 M;
(b) mixing the phosphate solution and the carbonate solution of said step (a) to prepare a mixed solution;
(c) adding a basic solution into the calcium salt solution of said step (a);
(d) adding a basic solution into the mixed solution of said step (b);
(e) mixing and reacting the calcium salt solution into which the basic solution has been added in said step (c) with the mixed solution into which the basic solution has been added in said step (d) to prepare a precipitate; and
(f) filtering, washing and drying the precipitate of said step (e) to obtain calcium phosphate,
wherein the calcium phosphate obtained in said step (f) has a primary particle size of 100 nm or less determined by SEM, and a specific surface area of 40 to 150 m²/g determined by BET method.

2. The method for preparing calcium phosphate of claim 1 **characterized in that**,
the calcium salt is one or more selected from the group consisting of Ca(NO₃)₂, Ca(OH)₂, CaCO₃, Ca(CH₃COO)₂, CaCl₂ and CaSO₄,
the phosphate is one or more selected from the group consisting of (NH₄)₂HPO₄, NH₄H₂PO₄, H₃PO₄, H₄P₂O, NaH₂PO₄ and KH₂PO₄, and
the carbonate is at least one selected from the group consisting of (NH₄)₂CO₃, NH₄HCO₃, K₂CO₃ and Na₂CO₃.

3. The method for preparing calcium phosphate of claim 1 **characterized in that**,
in said step (c), the basic solution is added such that the calcium salt solution of said step (a) has a pH of 8 to 12, and
in said step (d), the basic solution is added such that the mixed solution of said step (b) has a pH of 8 to 12.

4. The method for preparing calcium phosphate of claim 3 **characterized in that**,
the basic solution is one or more selected from the group consisting of NH₄OH, NaOH, KOH, LiOH and Mg(OH)₂.

5. The method for preparing calcium phosphate of claim 1 **characterized in that**,
in said step (f),
the precipitate of said step (e) is filtered with a filter, washed with distilled water or an organic solvent, and dried at a temperature of 50 to 100 °C.

## Patentansprüche

1. Verfahren zur Herstellung von Calciumphosphat **dadurch gekennzeichnet, dass** das Verfahren aufweist,
(a) Herstellen einer 0,5 bis 1,5 M Lösung eines Calciumsalzes, einer 0,3 bis 0,8 M Lösung eines Phosphats, und einer 0,1 bis 0,5 M Lösung eines Carbonats;
(b) Mischen der Phosphatlösung und der Carbonatlösung aus dem Schritt (a) zur Herstellung einer gemischten Lösung;
(c) Hinzufügen einer basischen Lösung zu der Calciumsalzlösung aus dem Schritt (a);
(d) Hinzufügen einer basischen Lösung zu der gemischten Lösung aus dem Schritt (b);
(e) Mischen und Reagieren der Calciumsalzlösung, in welche die basische Lösung in dem Schritt (c) hinzugegeben wurde, mit der gemischten Lösung, in welche die basische Lösung in dem Schritt (d) hinzugegeben wurde, um ein Präzipitat herzustellen; und
(f) Filtern, Waschen und Trocknen des Präzipitats aus dem Schritt (e), um Calciumphosphat zu erhalten,
wobei das Calciumphosphat, das in dem Schritt (f) erhalten wurde, eine durch SEM bestimmte vorherrschende Teilchengröße von 100 nm oder weniger aufweist, und eine durch das BET Verfahren bestimmte spezifische Oberfläche von 40 bis 150 m²/g aufweist.

2. Verfahren zur Herstellung von Calciumphosphat nach Anspruch 1 **dadurch gekennzeichnet, dass**
das Calciumsalz eines oder mehrere ausgewählt aus der Gruppe bestehend aus Ca(NO₃)₂, Ca(OH)₂, CaCO₃, Ca(CH₃COO)₂, CaCl₂ und CaSO₄ ist,
das Phosphat eines oder mehrere ausgewählt unter der Gruppe bestehend aus (NH₄)₂HPO₄, NH₄H₂PO₄, H₃PO₄, H₄P₂O, NaH₂PO₄ und KH₂PO₄ ist, und
das Carbonat mindestens eines ausgewählt aus der Gruppe bestehend aus (NH₄)₂CO₃, NH₄HCO₃, K₂CO₃ und Na₂CO₃ ist.

3. Verfahren zur Herstellung von Calciumphosphat nach Anspruch 1 **dadurch gekennzeichnet, dass**
in dem Schritt (c) die basische Lösung derart hinzugegeben wird, dass die Calciumsalzlösung aus dem Schritt (a) einen pH von 8 bis 12 aufweist, und
in dem Schritt (d) die basische Lösung derart hinzugegeben wird, dass die gemischte Lösung aus dem Schritt (b) einen pH von 8 bis 12 aufweist.

4. Verfahren zur Herstellung von Calciumphosphat nach Anspruch 3 **dadurch gekennzeichnet, dass**
die basische Lösung eines oder mehrere ausgewählt aus der Gruppe bestehend aus NH₄OH, NaOH, KOH, LiOH und Mg(OH)₂ ist.

5. Verfahren zur Herstellung von Calciumphosphat nach Anspruch 1 **dadurch gekennzeichnet, dass**
in dem Schritt (f), das Präzipitat aus dem Schritt (e) mit einem Filter filtriert, mit destilliertem Wasser oder einem organischen Lösungsmittel gewaschen, und bei einer Temperatur von 50 bis 100 °C getrocknet wird.

## Revendications

1. Procédé pour la préparation de phosphate de calcium **caractérisé en ce que** le procédé comprend,
(a) la préparation d'une solution de sel de calcium de 0,5 à 1,5 M, d'une solution de phosphate de 0,3 à 0,8 M, et d'une solution de carbonate de 0,1 à 0,5 M ;
(b) le mélange de la solution de phosphate et de la solution de carbonate de ladite étape (a) pour préparer une solution mélangée ;
(c) l'ajout d'une solution basique dans la solution de sel de calcium de ladite étape (a) ;
(d) l'ajout d'une solution basique dans la solution mélangée de ladite étape (b) ;
(e) le mélange et la mise en réaction de la solution de sel de calcium dans laquelle la solution basique a été ajoutée dans ladite étape (c) avec la solution mélangée dans laquelle la solution basique a été ajoutée dans ladite étape (d) pour préparer un précipité ; et
(f) la filtration, le lavage et le séchage du précipité de ladite étape (e) pour obtenir du phosphate de calcium,
le phosphate de calcium obtenu dans ladite étape (f) possédant une taille de particule primaire de 100 nm ou moins déterminée par SEM, et une superficie spécifique de 40 à 150 m²/grammes déterminée par le procédé BET.

2. Procédé pour la préparation de phosphate de calcium selon la revendication 1 **caractérisé en ce que**,
le sel de calcium est l'un ou plusieurs choisis dans le groupe constitué par Ca(NO₃)₂, Ca(OH)₂, CaCO₃, Ca(CH₃COO)₂, CaCl₂ et CaSO₄,
le phosphate est l'un ou plusieurs choisis dans le groupe constitué par (NH₄)₂HPO₄, NH₄H₂PO₄, H₃PO₄, H₄P₂O₇, NaH₂PO₄ et KH₂PO₄, et
le carbonate est au moins l'un choisi dans le groupe constitué par (NH₄)₂CO₃, NH₄HCO₃, K₂CO₃ et Na₂CO₃.

3. Procédé pour la préparation de phosphate de calcium selon la revendication 1 **caractérisé en ce que**,
dans ladite étape (c), la solution basique est ajoutée de sorte que la solution de sel de calcium de ladite étape (a) possède un pH de 8 à 12, et
dans ladite étape (d), la solution basique est ajoutée de sorte que la solution mélangée de ladite étape (b) possède un pH de 8 à 12.

4. Procédé pour la préparation de phosphate de calcium selon la revendication 3 **caractérisé en ce que**,
la solution basique est l'une ou plusieurs choisies dans le groupe constitué par NH₄OH, NaOH, KOH, LiOH et Mg(OH)₂.

5. Procédé pour la préparation de phosphate de calcium selon la revendication 1 **caractérisé en ce que**,
dans ladite étape (f),
le précipité de ladite étape (e) est filtré avec un filtre, lavé avec de l'eau distillée ou un solvant organique, et séché à une température de 50 à 100 °C.
